Europäisches Patentamt

European Patent Office

Office européen des brevets

(11) Numéro de publication: **0 262 010**
**A1**

## (12) DEMANDE DE BREVET EUROPEEN

(21) Numéro de dépôt: **87401929.2**

(22) Date de dépôt: **25.08.87**

(51) Int. Cl.4: **C 12 P 13/04**
C 12 P 7/62, C 12 P 41/00

(30) Priorité: **28.08.86 FR 8612184**

(43) Date de publication de la demande:
**30.03.88 Bulletin 88/13**

(84) Etats contractants désignés:
**AT BE CH DE ES FR GB IT LI LU NL SE**

(71) Demandeur: **INSTITUT DE RECHERCHES CHIMIQUES ET BIOLOGIQUES APPLIQUEES (I.R.C.E.B.A.) Société à responsabilité limitée dite:**
**62, Grande-Rue**
**F-78490 Vicq (FR)**

(72) Inventeur: **Morinière, Jean-Luc**
**43, Avenue Dr. Arnold Netter**
**F-75012 Paris (FR)**

**Danrée, Bernard**
**69 Bis Boulevard Devaux**
**F-78300 Poissy (FR)**

(74) Mandataire: **Combe, André et al**
**CABINET BEAU DE LOMENIE 55, rue d'Amsterdam**
**F-75008 Paris (FR)**

(54) Procédé d'estérification stéréospécifique d'un mélange d'acides aminés par réaction dudit mélange en solution dans l'alcool d'estérification en présence de papaine.

(57) La présente invention concerne un procédé d'estérification stéréospécifique d'un mélange d'acides aminés naturels et xénobiotiques dont l'un est sous la forme L et l'autre sous la forme D, caractérisés en ce que :
- l'estérification est effectuée sur des aminoacides protégés sur la fonction aminée, lesdits aminoacides protégés étant en solution dans l'alcool d'estérification,
- l'estérification est effectée en présence d'une faible quantité d'eau et à un pH compris entre environ 3 et 6,
- et on opère en présence de papaïne.

EP 0 262 010 A1

## Description

### Procédé d'estérification stéréospécifique d'un mélange d'acides aminés par réaction dudit mélange en solution dans l'alcool d'estérification en présence de papaïne

Les aminoacides naturels du type méthionine, alanine..., et xénobiotiques du type phénylalanine substituée se présentent souvent sous la forme d'un mélange sensiblement équimoléculaire de deux forms : la forme L et la forme D. Par ailleurs, on sait que pour un grand nombre d'applications il est souhaitable de pouvoir disposer desdits aminoacides sous une forme pure soit L, soit D.

Il est donc souhaitable de disposer d'un moyen pratique et industriel permettant, à partir d'un mélange desdites formes L et D, de préparer une forme énantiomérique pure de l'aminoacide.

On a déjà proposé un certain nombre de moyens permettant la séparation des formes L et D d'aminoacide à partir d'un mélange de ces formes ; la présente invention vise un moyen nouveau pour réaliser cette séparation, ledit moyen présentant l'avantage d'une grande simplicité, donc d'une industrialisation facile.

Le moyen préconisé dans la présente invention a pour base le fait d'effectuer une estérification stéréospécifique du mélange des aminoacides. L'estérification sera dite stéréospécifique si elle ne porte que sur l'une des formes optiquement actives (L ou D) de l'aminoacide racémique.

On a déjà décrit, par exemple, dans le brevet français 83 01839, un procédé pour réaliser une estérification stéréospécifique d'acide aminé dans lequel on effectue une estérification dudit acide aminé en présence d'un enzyme dans un milieu dydro-organique. Mais le procédé décrit dans ce brevet est difficile à mettre en oeuvre en particulier du fait que l'enzyme choisi est l'$\alpha$-chymotrypsine et que l'on travaille dans un milieu biphasique chloroforme-eau.

La présente invention vise un moyen surmontant les difficultés rencontrées dans le brevet ci-dessus mentionné et permettant ainsi la réalisation industrielle d'une estérification stéréospécifique.

Ainsi, la présente invention concerne un procédé d'estérification stéréospécifique d'un mélange D-L d'acide aminé caractérisé en ce que :

- ladite estérification est réalisée en un milieu homogène, qui est un solvant desdits acides aminés et qui est constitué essentiellement de l'alcool d'estérification, sur un mélange d'acides aminés dans lesquels la fonction amine a été protégée,

- l'estérification est effectuée en présence d'une quantité d'eau comprise entre 2 équivalents pour 1 équivalent d'aminoacide et 2,5 équivalents pour 1 équivalent d'aminoacide et à un pH compris entre environ 3 et environ 6,

- la réaction d'estérification est réalisée en présence de papaïne.

L'estérification est réalisée en utilisant comme produit de départ un aminoacide se trouvant sous ses deux formes L et D.

Ces deux aminoacides ont été protégés sur leur fonction amine par des radicaux connus pour réaliser ladite protection lorsque l'on effectue des réactions sur lesdits aminoacides. Ainsi, on peut utiliser pour réaliser cette protection des radicaux benzyloxycarbonyle, t-butoxycarbonyle ou fluorénométhyloxycarbonyle. Ces radicaux ont été cités par ordre d'intérêt décroissant et c'est ainsi que le radical préféré est le benzyloxycarbonyle.

L'estérification est réalisée dans un milieu homogène qui est constituée de l'alcool d'estérification, ledit alcool étant un solvant des deux aminoacides et étant un alcool aliphatique primaire linéaire ou ramifié ayant de 1 à 8 atomes de carbone.

L'estérification a lieu en présence d'une failble quantité d'eau. Il a été prouvé en effet qu'en absence totale d'eau (par exemple en opérant dans un alcool pur) la réaction ne se fait pas ou se fait mal. La quantité d'eau utilisée sera d'au moins environ 2 équivalents pour 1 équivalent d'aminoacide mais sera inférieure à environ 2,5 équivalents pour 1 équivalent d'aminoacide.

La réaction d'estérification est réalisée en présence de papaïne ; par papaïne on entend non seulement le produit pur rencontré et vendu sous le nom de papaïne mais encore les produits industriels qui, comme on le sait, contiennent des quantités notables de chymopapaïne et éventuellement certaines impuretés qui n'ont pas d'influence notable sur le déroulement de la réaction.

Les quantités de papaïne utilisées peuvent être très variables ; on emploiera au minimum environ 5% en poids de papaïne par rapport aux aminoacides présents ; mais, dans une certaine concentration, la vitesse de la réaction étant croissante au fur et à mesure que l'on augmente, toutes choses égales par ailleurs, la proportion de papaïne, on peut avoir intérêt à utiliser des poids de papaïne sensiblement égaux à ceux des acides aminés présents.

Comme dans de nombreuses réactions enzymatiques utilisant la papaïne il peut être souhaitable d'opérer en présence d'un activateur - connu - de cette papaïne ; on emploiera par exemple de faibles quantités de chlorhydrate de cystéine ou de chlorhydrate de cystéamine.

Les conditions de la réaction sont mentionnées ci-après :

- la concentration des aminoacides dans l'alcool sera, pour des questions de rendement à l'unité de volume, aussi élevée que possible tout en restant dans les limites de solubilité desdits aminoacides dans l'alcool utilisé. Il a été trouvé qu'une concentration en aminoacides de 0,6 à 0,9 mol/l était souhaitable,

- l'estérification devra se dérouler à un pH compris entre environ 3 et environ 6 ; il a été constaté en effet qu'en dehors de ces limites le rendement de la réaction (c'est-à-dire le % de l'aminoacide de forme L qui a été stéréospécifiquement estérifié) était très faible et inférieur à environ 10%. Il existe pour chaque aminoacide et pour chaque alcool un pH compris entre 4 et 5,5, pour lequel le rendement est optimum. La réalisation des pH souhaitable peut être faite par utilisation de tout mélange tampon adéquat comme par exemple les mélanges de citrate de sodium et d'acide citrique. Bien évidemment, l'eau contenue dans les mélanges tampons devra être prise en compte dans la faible quantité d'eau nécessaire à l'obtention d'une bonne réaction,

- a la fin de la réaction d'estérification stéréospécifique selon l'invention, on obtiendra dans la solution u mélange sensiblement équimoléculaire d'ester de l'acide aminé protégé sous forme L et d'acide aminé protégé sous forme D et des particules de papaïne en suspension. Il sera alors facile au spécialiste de récupérer, après filtration de la papaïne, l'ester protégé d'une part et l'aminoacide protégé d'autre part puis de transformer et déprotéger les produits restants pour obtenir les aminoacides sous les formes L et D. Les rendements de l'estérification stéréospécifique réalisée peuvent être très élevés et atteindre 97 - 98 % par rapport à l'aminoacide de forme L initialement présent.

Les exemples non limitatifs ci-après illustrent l'invention.

Exemple 1 : Synthèse de l'ester éthylique de l'acide (N-benzyloxycrabonyl)-L-Alanine :

Dans un tricol de 250 ml équipé d'une agitation magnétique, d'un thermomètre et d'un réfrigérant à eau, on introduit :
- 60 ml d'éthanol absolu
- 10,68 g (0,0479 mol) de (N-benzyloxycarbonyl)-DL-Alanine puis à la solution obtenue :
- 1 ml de tampon citrate à pH : 4,5 qui contient 0,12 g de chlorhydrate de cystéine.
- 1 ml de tampon citrate dissous dand 20 ml d'éthanol absolu.

Cette solution sert à rincer le récipient utiliseé à l'addition précédente.

Le milieu réactionnel homogène est porté à 50°C ; à cette température, on introduit :
- 1,33 g de papaïne (à 3,6 UI/mg) sous bonne agitation.

La réaction d'estérification est suivie en CLHP [colonne O.D.S. Hypersil 250 mm, 5 μ (SFCC) ; solvant MeOH/H$_2$O/NH$_4$OH conc. = 67/30/3 ; V$_R$ = 4,95 : 5,05 ml] ou en CPG [(colonne OV 101 5 % ; 1,8 m) ; Inj. = Det. = 300°C ; Programmation ; 100 - 290°C/8°C/min ; t$_R$ : 11,2 min].

La réaction n'évolue plus qu-delà de 24 h à la température de 50°C.

On refroidit et filtre la papaïne. La phase alcoolique limpide est concentrée à sec à l'évaporateur rotatif. Le résidu est repris par 150 ml de chloroforme ; la phase organique est lavée par 2 × 100 ml de bicarbonate de sodium à 5 % puis à l'eau jusqu'à neutralité.

La solution chloroformique est séchée sur sulfate de magnésium et concentrée à sec.

On obtient ainsi 5,82 g (0,0232 mol) de l'ester éthylique de l'acide (N-benzyloxycarbonyl)-L-Alanine (Rendement : 97,1 %).

L'ester obtenu est une huile qui cristallise lentement (F. 30°C) dont le pouvoir rotatoire dans le méthanol est :

[α]   = - 32° ± 2°, (c = 1).

La phase aqueuse basique de lavage de la solution chloroformique (200 ml) est acidifiée à froid par l'acide chlorhydrique 6 N jusqu'à pH 2. Le précipité est filtré et lavé sur le filtre par 2 × 100 ml d'eau puis séché à l'étuve sous vide à 60°C [m : 4,53 g (0,0203 mol), F.°C : 86 - 87°C ; [α]   = + 12,5° ± 1,2° (c = 2, AcOH) ; ee = 90 %].

L'acide (N-benzyloxycarbonyl)-D-Alanine est purifié par cristallisation dans 10 volumes d'un mélange chloroform-éther de pétrole (30-70). On obtient ainsi 4,3 g (0,0193 mol) [rendement pondéral global : 81 %] d'un produit pur dont le point de fusion (mesuré au banc Köfler) est de 87 - 88°C et dont le pouvoir rotatoire dans l'acide acétique est :
[α]   = + 13,9° ± 1,2° (c = 2).

Exemple 2 : Synthèse de l'ester méthylique de l'acide (N-benzyloxycarbonyl)-L-Alanine :

Dans un réacteur de 2 l équipé d'une agitation efficace, d'un thermomètre et d'un réfrigérant à reflux, introduire :
-1000 ml de méthanol
- 200 g (0,897 mol) de (N-benzyloxycarbonyl)-DL-Alanine
- 38 ml de tampon citrate à pH : 4,5 qui contient 2,24 g de chlorhydrate de cystéamine
- 200 ml de méthanol qui servent à rincer l'ampoule d'introduction.

Le milieu réactionnel homogène est porté à 50°C sous bonne agitation ; on introduit alors :
- 25 g de papaïne à 50 nKat/mg.

L'avancement de la réaction est suivi selon l'exemple 1 [CLHP : V$_R$ : 4,15 - 4,20 ml : CPG : t$_R$ = 9,8 min] ; après 24 h, le traitement décrit précédemment conduit à 90,5 g (0,382 mol) d'ester méthylique de l'acide (N-benzyloxycarbonyl)-L-Alanine (rendement 85,2 %) dont le point de fusion (Köfler) est de 44 - 46°C et dont le pouvoir rotatoire dans le méthanol est :
[α]   = - 33° ± 2° (c = 1).

Selon l'exemple 1, après traitement des phases basiques de lavage et cristallisation, on obtient 80 g (0,359 mol) d'acide (N-benzyloxycarbonyl)-D-Alanine [Rendement : 80 % ; F.°C : 87 - 88°C; [α]   = + 13,8° ± 1,2°, (c = 2,AcOH)].

**Exemple 3:**

En opérant comme ci-dessus et en changeant l'alcool d'estérification on a pu obtenir les produits suivants :

A) l'ester n-propylique de l'acide (N-benzyloxycarbonyl)-L-Alanine avec un rendement de 75 %, dont le point de fusion est de 30 - 32°C et le pouvoir rotatoire dans le méthanol est :

$[\alpha]$ = -27,9° ± 2° (c = 1).

B) l'ester n-butylique de l'acide (N-benzyloxycarbonyl)-L-Alanine avec un rendement de 80 %, dont le pouvoir rotatoire dans le méthanol est :

$[\alpha]$ = -25° ± 2° (c = 1).

**Exemple 4 :**

En opérant en présence d'alcool éthylique absolu comme dans l'exemple 1, on a pu obtenir les produits suivants :

C) l'ester éthylique de l'acide (t-butoxycarbonyl)-L-Alanine avec un rendement de 73 %, [CPG : $t_R$ = 3 min], dont le pouvoir rotatoire dans le méthanol est :

$[\alpha]$ = -41,5° ± 2,4° (c = 1)

D) l'ester éthylique de l'acide (N-fluorénométhyloxycarbonyl)-L-Alanine avec un rendement de 35 %, dont le point de fusion est de 109 - 111°C, [CPG : $t_R$ = 8 min ; CLHP : $V_R$ = 13,6 - 13,8ml] et le pouvoir rotatoire dans le méthanol est :

$[\alpha]$ = -18° ± 2° )c = 1).

**Exemple 5 : Synthèse de l'ester éthylique de l'acide (N-benzyloxycarbonyl)-L-Phénylalanine**

Dans un tricol de 250 ml équipé d'une agitation magnétique, d'un thermomètre et d'un réfrigérant à eau, on introduit :

- 40 ml d'éthanol absolu
- 7,15 g (0,0239 mol) de (N-benzyloxycarbonyl)-DL-Phénylalanine
- 1 ml de tampon citrate pH : 4,5 qui contient 0,06 g de chlorhydrate de cystéine
- 0,89g de papaïne à 3,0 UI/mg dans les conditions opératoires décrites dans les exemples 1 et 2.

Au bout de 24 h de réaction à 50°C (CPG : $t_R$ = 17,6 min, CLHP : $V_R$ = 9,7 ml) le milieu réactionnel est traité selon les exemples précédents.

On obtient ainsi 3,40 g (Rendement 87 % ; 0,0104 mol) de l'ester éthylique de l'acide (N-benzyloxycarbo-nyl)-L-Phénylalanine dont le pouvoir rotatoire dans le méthanol est :

$[\alpha]$ = - 12,7° ± 1,2° (c = 1).

L'acide (N-benzyloxycarbonyl)-D-Phénylalanine est récupéré avec un rendement global de 82 % (m = 2,93 g ; 9,80 mmol) dont le point de fusion est de 90 - 91°C et dont le pouvoir rotatoire dans l'éthanol absolu est :

$[\alpha]$ = - 5,8° ± 1,2° (c = 2).

**Exemple 6 :**

En opérant comme ci-dessus, on a pu obtenir les produits suivants, dont les constantes physiques sont résumées dans le tableau ci-après :

X = Aminoacide L

Z = Benzyloxycarbonyl-

| X | Rendement | $[\alpha]_D^{20}$ (1, MeOH) | F.°C$_{BK}$ | HPLC ml |
|---|---|---|---|---|
| Val | 38 | − 7,6° | --- | 7,7 |
| Leu | 90 | − 26,5° | --- | 11,4 |
| Met | 95 | − 29,1° | 33–35 | 6,36 |
| Tyr | 95 | − 7,4° | 78–80 | 4,4 |
| Ser | 53 | − 12,2° | --- | 4,2 |
| Trp | 92 | − 9,9° | 85–87 | 11,8 |
| Asp | 63 | − 19,4° | 84–86 | 1,9 |
| Gln | 89 | − 19,8° | 116–118 | 3,6 |
| Dichloro-3,4-Phe | 91 | − 12° | 67–69 | 22,6 |
| Fluoro-4-Phe | 82 | − 11,6° | 42–44 | 10,6 |
| Nitro-4-Phe | 75 | − 22,1° | 91–93 | 7,5 |
| Nitro-3-Tyr | 67 | − 4,8° | 85–87 | 1 |
| Nor-Leu | 97 | − 18,5° | --- | 12,4 |
| Nor-Val | 98 | − 25,2° | --- | 9,0 |
| Amino-2-Butyroyle | 90 | − 25,2° | --- | 6,2 |
| Amino-2-Octanoyle | 57 | − 18,3° | --- | 27,5 |
| Thényl-Gly | 42 | + 39,4° | 46–48 | 9,5 |

Dans le tableau ci-dessus, F°C$_{BK}$ indique la température de fusion du produit obtenu mesurée, en degrés centigrages, au banc Köfler ; les lettres HPLC signifient "Haute Pression Liquide Chromatographie".

## Revendications

1. Procédé d'estérification stéréospécifique d'un mélange D-L d'acide aminé en présence d'un enzyme caractérisé en ce que :
- ladite estérification est réalisée en un milieu homogène, qui est un solvant desdits acides aminés et qui est constituée essentiellement de l'alcool d'estérification, sur un mélange d'acides aminés dans lesquels la fonction amine a été protégée,
- l'estérification est effectuée en présence d'une quantité d'eau comprise entre 2 équivalents pour 1 équivalent d'aminoacide et 2,5 équivalents pour 1 équivalent d'aminoacide et à un pH compris entre environ 3 et environ 6,
- la réaction d'estérification est réalisée en présence de papaïne.

2. Procédé selon la revendication 1, caractérisé en ce que la papaïne utilisée est une papaïne industrielle.

3. Procédé selon l'une des revendications 1 et 2, caractérisé en ce que la fonction amine a été protégée par un radical benzyloxycarbonyle.

4. Procédé selon l'une des revendications 1 à 3, caractérisé en ce que la quantité de papaïne utilisée sera comprise entre 5 et 100 % environ du poids des acides aminés utilisés.

5. Procédé selon l'une des revendications 1 à 4, caractérisé en ce que la concentration des acides aminés dans l'alcool d'estérification est comprise entre 0,6 et 0,9 mol/l.

| | Office européen des brevets | RAPPORT DE RECHERCHE EUROPEENNE | Numero de la demande |
|---|---|---|---|

EP 87 40 1929

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 97, no. 19, 8 novembre 1982, page 349, no. 158940u, Columbus, Ohio, US; F.L. PINHEIRO-DA-SILVA et al.: "Papain-catalyzed esterification of N-tert-butyloxycarbonyl and N-benzyloxycarbonyl amino acids" & BRAZ. J. MED. BIOL. RES. 1981, 14(6), 373-7 <br>--- | 1 | C 12 P 13/04 <br> C 12 P 7/62 <br> C 12 P 41/00 |
| Y | FR-A-2 540 515 (PIERRE FABRE) <br> * Revendications * <br>--- | 1 | |
| Y | BULLETIN DE LA SOCIETE CHIMIQUE DE FRANCE, vol. II, 1980, pages 76-79; D. TARQUIS et al.: "Synthèse enzymatique d'esters d'acides aminés en milieu organique" <br> * Pages 76-79, en entier * <br>--- | 1 | |
| Y | PATENT ABSTRACTS OF JAPAN, vol. 7, no. 273 [1418], décembre 1983, section C, page 96 C 198; & JP-A-58 152 856 (SAGAMI CHUO KAGAKU KENKYUSHO) 10-09-1983 <br>--- | 1 | DOMAINES TECHNIQUES RECHERCHES (Int. Cl.4) <br><br> C 12 P |
| Y | CHEMICAL ABSTRACTS, vol. 69, no. 7, 12 août 1968, page 2309, no. 24806t, Columbus, Ohio, US; G. TALSKY et al.: "Temperature-dependence of enzymic reactions. V. Hydrolysis of N-benzoyl-L-arginine ethyl ester by papain" & Z. NATURFORSCH., B 1968, 23(5), 645-9 <br>----- | 1 | |

Le présent rapport a été établi pour toutes les revendications

| Lieu de la recherche | Date d'achèvement de la recherche | Examinateur |
|---|---|---|
| LA HAYE | 10-12-1987 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)